Europäisches Patentamt

European Patent Office

Office européen des brevets

(11) Publication number: 0 369 176 A2

# EUROPEAN PATENT APPLICATION

(21) Application number: 89119250.2

(22) Date of filing: 17.10.89

(51) Int. Cl.5: G01N 33/543, G01N 21/17

(30) Priority: 19.10.88 JP 261539/88

(43) Date of publication of application:
23.05.90 Bulletin 90/21

(84) Designated Contracting States:
CH DE FR GB IT LI

(71) Applicant: HITACHI, LTD.
6, Kanda Surugadai 4-chome Chiyoda-ku
Tokyo(JP)

Applicant: Hitachi Instrument Engineering
Co., Ltd.
882, Ichige
Katsuta-shi Ibaraki-ken(JP)

(72) Inventor: Imai, Kyoko
Tsukubadai Apartment 1-103 663, Ichige
Katsuta-shi(JP)
Inventor: Nomura, Yasushi
991-15, Motoyoshidacho
Mito-shi(JP)
Inventor: Makiguchi, Hiroko
622-22, Ichige
Katsuta-shi(JP)

(74) Representative: Dost, Wolfgang,
Dr.rer.nat.,Dipl.-Chem. et al
Patent- & Rechtsanwälte Bardehle .
Pagenberg . Dost . Altenburg . Frohwitter .
Geissler & Partner Galileiplatz 1 Postfach 86
06 20
D-8000 München 86(DE)

(54) Method for immunoassay using photoacoustic spectroscopy.

(57) In this invention, antigen-antibody complex is labeled with a color material and photoacoustic properties of the label are determined by photoacoustic spectroscopy. As the color material, a fluorescent substance, a dye or colored particles can be used.

The immune reaction of the labeled antigen or antibody is carried out in liquid under the condition that the solid phase is present. As the result of immune reaction, the labeled antigen-antibody complex is immobilized on the surface of the solid phase. Then, the solid phase is separated from the liquid and moved to the position for exposure to light and intermittently irradiated by the light. An acoustic signal given thereby is detected. According to the present invention, a pressure medium for photoacoustic determination is a gas. The method permits to detect antigen or antibody with high sensitivity.

FIG. 1

## METHOD FOR IMMUNOASSAY USING PHOTOACOUSTIC SPECTROSCOPY

### BACKGROUND OF THE INVENTION

#### FIELD OF THE INVENTION:

The present invention relates to an immunoassay method and more particularly, to a method for immunoassay which is suitable for analyzing and determining the immune component in a sample utilizing a photoacoustic spectroscopy.

#### STATEMENT OF THE RELATED ART:

For determination of an antigen or antibody contained in body fluids with high sensitivity, attention has recently been brought to a method for immunoassay using a photoacoustic spectroscopy. This method is described, for example, in Japanese Patent Application KOKAI (Laid-Open) No. 63-44149. According to the prior art, a particulate antigen-antibody complex is formed in liquid and the resulting dispersion of the complex is introduced into a photoacoustic cell, where photoacoustic measurement is made therein. In this case, utilizing particle diameter-dependent sensitivity in photoacoustic spectroscopy, the sensitivity of a photoacoustic spectrometer is rendered highly sensitive to the particulate substance to be analyzed, using an exited light having a wavelength either identical with or similar to the size of the particulate substance to be analyzed, whereby the particulate substance is selectively detected and quantitatively determined.

An invention of U.S. Serial No. 283,814 filed December 13, 1988 also relates to immunoassay using photoacoustic spectroscopy. In the prior invention, an immune reaction is caused within a reactor to form an immune complex labeled with fine particles on the solid phase. Then, the fine particles are separated from the solid phase in the reactor and the dispersion of the fine particles is introduced into a measuring cell for photoacoustic spectroscopy. The fine particles in the dispersion are measured.

According to Japanese Patent Application KOKAI No. 63-44149 supra, the particulate antigen-antibody complex can be detected in such a state that influence by other particles is minimized. However, the concentration of rheumatoid factor, cancer specific antigen, etc. is extremely low and it is thus desired to develop a method for measurement with much higher sensitivity. The prior invention described above is also desired to achieve measurement with much higher sensitivity.

### SUMMARY OF THE INVENTION

An object of the present invention is to provide a method for immunoassay which permits to measure an antigen or antibody contained even in a trace amount in body fluids with high sensitivity, utilizing a photoacoustic spectroscopy.

According to the present invention, the antigen-antibody complex is labeled with a color material and photoacoustic properties of the label are determined by photoacoustic spectroscopy. As the color material, a fluorescent substance, a dye or colored particles can be used.

The immune reaction of the labeled antigen or antibody is carried out in liquid under the condition that the solid phase is present. As the result of immune reaction, the labeled antigen-antibody complex is immobilized on the surface of the solid phase. Then, the solid phase is separated from the liquid and moved to the position for exposure to light and intermittently irradiated by the light. An acoustic signal given thereby is detected. According to the present invention, a pressure medium for photoacoustic determination is a gas.

In a photoacoustic spectroscopy, it is essential that a substance absorbs light. By absorbing light, the substance takes in light energy, and atoms or molecules constituting the substance are excited. Owing to the light energy which the atoms or the molecules have taken in, the atoms or the molecules release heat energy when they return to the ground state. In this process, an acoustic wave is generated by the heat energy. According to the photoacoustic spectroscopy, thus generated acoustic wave is detected.

A photoacoustic spectrometer comprises as main constitutional elements a laser light source, a chopper for intermitting a light, a photoacoustic cell, an acoustic sensor, an amplifier and a signal processor system. After light from the light source has passed through a photometer, the light is converted to intermittent light by means of the chopper to be casted on the photoacoustic cell. In the cell, a sample absorbs the intermittent light, whereby a photoacoustic signal is generated. The photoacoustic signal is detected by the acoustic sensor such as a microphone.

In a preferred embodiment of the present invention, a solid phase to be determined is placed in the photoacoustic cell which is sealed in such a state that gas is present, and a leak into the gas of heat generated as a result of the light absorption by a sample is detected as a periodic pressure change by means of a highly sensitive microphone.

A pressure change (photoacoustic signal) generated when a sample is exposed to an incident light is converted into an electric signal (generation of voltage) by means of a microphone or a piezoelectric element. By previously preparing a calibration curve between the concentration of antigen and the photoacoustic signal converted into electric signal using an antigen having known concentrations, the antigen-antibody reaction can be quantitatively analyzed by a photoacoustic spectroscopy, whereby the desired antigen or antibody can be quantitatively determined.

According to a preferred embodiment of the present invention, trace components which could only be determined from a practical viewpoint by radioimmunoassay (RIA) heretofore can be quantitatively determined rapidly with high sensitivity in a simple manner.

Further by using a plurality of species in the standard substance, a plurality of analyses can also be determined concurrently.

BRIEF DESCRIPTION OF THE DRAWINGS

Fig. 1 shows an outline of the whole construction of a photoacoustic apparatus for practicing an embodiment of the present invention.

Fig. 2 shows an example of calibration curve for determination of HCG.

Fig. 3 shows a calibration curve for concurrent determination of AFP and CEA.

Fig. 4 shows an example of a calibration curve for determination of TSH.

Fig. 5 shows a pretreatment equipment within the analytical apparatus shown in Fig. 1.

Fig. 6 shows a cross sectional construction of measuring cell used in the apparatus of Fig. 1.

DETAILED DESCRIPTION OF THE PREFERRED EMBODIMENTS

In a preferred embodiment of the present invention, a first antibody immobilized to the solid phase is reacted with a labeled second antibody capable of specifically reacting with the first antibody. By this reaction, the immune complex labeled with a color material is formed on the surface of the solid phase together with the antibody-bound antigen contained in the sample. The solid phase is separated from the liquid, introduced into a measuring cell and measured by a photoacoustic spectroscopy. In this case, the second antibody labeled with a label is not required to be specifically reactive with the antigen to be analyzed but is sufficient to specifically react with the first antibody. For example, when the analyte is α-feto-protein (AFP) and the first antibody is rabbit-anti-AFP, sheep anti-rabbit IgG antibody can be used as the second antibody. For this reason, it can be avoided to use expensive antibody in large quantities. In view of reagent costs, this embodiment is thus excellent.

As the label, there may be advantageously used a dye, a fluorescence emitting substance, etc. which can be bound to the analyte or antibody capable of specifically reacting with the analyte. Furthermore, a dye or a fluorescent substance included in or bound or adsorbed to microspheres such as liposome; a dye or a fluorescence bound or adsorbed to a carrier such as latex particles; colorless particles or carriers which are labeled with colored latex, etc. may also be used as the label. These substances are often referred to as color materials hereinafter.

The solid phase preferably takes a portable structure formed into a film or plate having a surface capable of holding liquid therein, like a microplate which becomes a little bit hollow; a film made of synthetic resin such as nylon, polyester, etc.; a container packed with a filler such as agarose, Sepharose, silica gel, etc. As the solid phase, glass pieces or paper which can be immersed in liquid, and the like may also be used.

An embodiment using a plate for thin layer chromatography (TLC) as the solid phase is described below.

In measurement of a solid sample, i.e., reacted solid phase, by photoacoustic spectroscopy, a substance present around the surface can be measured with relatively high sensitivity. This is because, when a photoacoustic signal is detected by means of a microphone using as a pressure medium, e.g., gas, a substance such as an adsorbed matter, etc. present around the surface of the solid phase can be detected with high sensitivity, since only the region around the surface of the solid sample contributes to the signal. Where solid phase itself which is a substrate substantially has no absorption and has a larger reflectivity, this tendency is more remarkable. In TLC, silica gel, alumina or the like is often generally used as the adsorbent. These substances are optically opaque and powdery, so that quantitative determination of the labeled reaction product adsorbed by optical means such as ordinary transmission method or reflection method results in poor accuracy in measurement due to scattered light and in insufficient measurement sensitivity. For this reason, quantitative or qualitative determination is generally performed by firstly exposing a TLC plate after development to UV light or applying iodine gas to confirm the position of spot, then scratching the spot off and extracting the adsorbed matter with an appropriate solvent, and then measuring its absorption spectrum, etc. Therefore, a very long period of time is required for the determination.

According to the photoacoustic spectroscopy, however, positioning of the spot and quantitative or qualitative determination of the adsorbed matter can be made in the adsorbed state. In this case, since background noises derived from the adsorbent affect the detection limit, the adsorbed matter, i.e., the label is colored so that S/N can be greatly improved.

As compared to the conventional absorbance measurement method, the present invention permits to measure with sensitivity as high as two figures or more in such a state that the label is adsorbed on the reacted solid phase.

When a color material is used as the label, a ratio of signal to background noises (S/N) for photoacoustic measurement is improved. The solid phase on which the antigen-antibody complex labeled with the color material is adsorbed is moved to the position of a spectrometer for exposure to light. When the solid phase is exposed to a laser light, the color material present on the surface of the solid phase absorbs the light and releases heat energy in response to the light absorbed. The greater the quantity of light absorbed, the greater the photoacoustic signal obtained. As a color of the label, it is chosen to have the absorption maximum substantially in compliance with the central wavelength of the excited monochromatic light. For example, when an argon laser having a wavelength of 488 nm is used as the excited light, Food Yellow 3 having the absorption maximum wavelength of 482 nm is used as a dye for the label; in this case, good results are obtained.

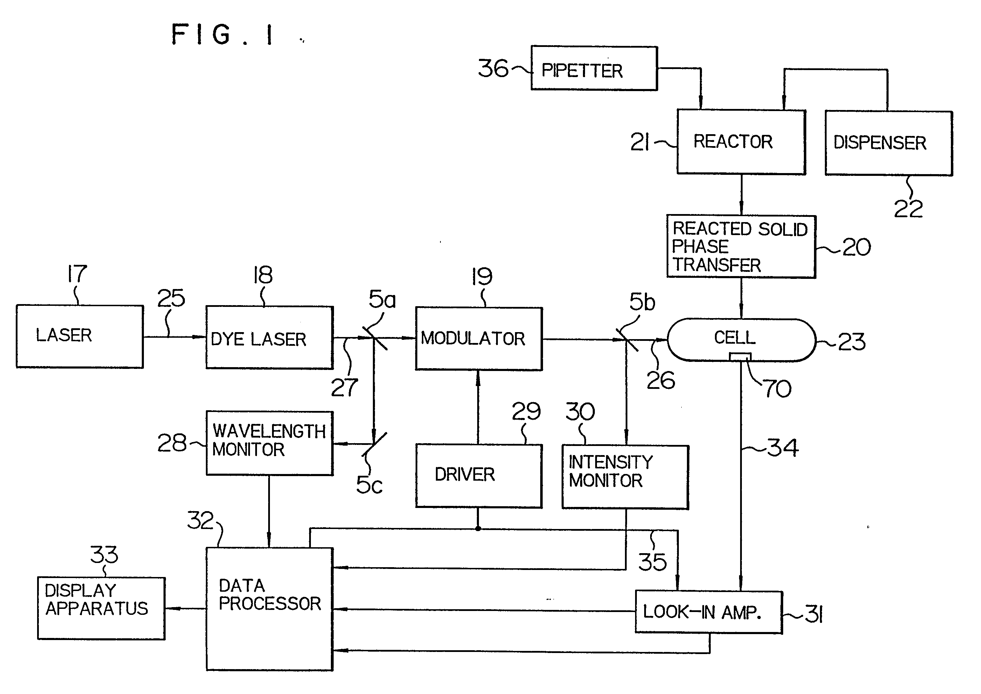

An outlined construction of an analytical apparatus for practicing one embodiment of the present invention is shown in Fig. 1.

Light source for excitation 17 is a light source for argon laser having an output of 10W. Laser light 25 from argon laser light source has oscillation rays in several wavelengths at 488 nm, 514.5 nm and over the ultraviolet to visible regions. Depending upon the size of the reaction product to be analyzed, an appropriate dye in dye laser 18 is subjected to pumping. For example, when the size of the reaction product is 0.6 to 0.7 $\mu$m, the wavelength of the excited light 25 from the argon laser light source 17 is set at 514.5 nm and rhodamine is used as the dye for the dye laser 18. The laser light 27, its wavelength being modified in response to the size of the analyte, is in part divided by half mirror 5a. The wavelength of incident light via reflection mirror 5c is confirmed by wavelength monitor 28. The remaining laser light is modulated to a periodic rectangular wave by a modulator 19 comprising light chopper of rotary blade type and is converted into the excited light 26. The excited light 26 is cast on a measuring cell 23. In the cell, the sample absorbs the light to give a photoacoustic signal. The photoacoustic signal 34 generated in the measuring cell 23 is detected by means of a microphone or piezoelectric element 70 and amplified by a lock-in amplifier 31, by referring to reference signal 35 synchronized with light intensity modulation output from a driver 29 of the modulator 19. A part of the excited light 26 is divided by the half mirror 5b and its intensity is monitored by a light intensity monitor 30. Information on the intensity of the photoacoustic signal through line 72 and the phase of the photoacoustic signal through line 73 is input to a data processor 32; further information on the wavelength, modulated frequency and intensity of the excited light from a wavelength monitor 28, the modulation driver 29 and the light intensity monitor 30 is also input to the data processor 32 through line 71. The data processor 32 displays parameters on conditions for the measurement, for example, information on wavelength of the excited light, modulation frequency, etc. on a display apparatus 33 or performs data processing of the measurement results and displays a calibration curve of the reaction product or quantitative results on a sample having unknown concentration, and the like on the display apparatus 33.

Body fluids such as plasma are placed with a sample pipetter 36 on a disk-like apparatus within a reactor 21. Reagent solution for forming the antigen-antibody complex is added through a reagent dispenser 22. The reacted solid phase on which the immune complex is formed in the reactor 21 is inserted into the photoacoustic cell 23 by means of a reacted solid phase transfer 20. The cell is sealed in such a state that the air is present. The measuring cell 23 consists of a glass container equipped with a light incident window, inside of which a microphone is placed. An open-close cover provided in the cell is closed and sealed when the reacted solid phase is encased in the cell.

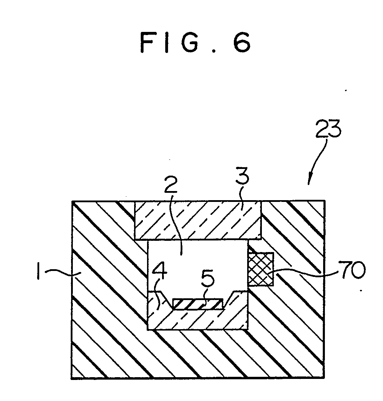

An example of the construction of the measuring cell 23 used in the apparatus shown in Fig. 1 is shown in Fig. 6. The cell 23 has a cylindrical room, the upper part of which can be opened by a reactor forming material 1. The room 2 is sealable with a quartz glass-made cover 3. The microphone 70 is embedded in the side wall of the room 2. A stand 4 of transparent glass is mounted to the bottom of the room. The disk-like solid phase 5 can be placed at the center of the stand 4. In the example, the solid phase has a size of 10 mm in diameter and 2 mm in high. However, the solid phase can be changed to various sizes. When the room is covered, the volume of the space is 0.5 to 1.0 ml. The excited light is cast on the solid phase 5 through the cover 3. Therefore, the cover 3 functions as a light incident window. For the construction of such measuring cell 23, there are various modifications.

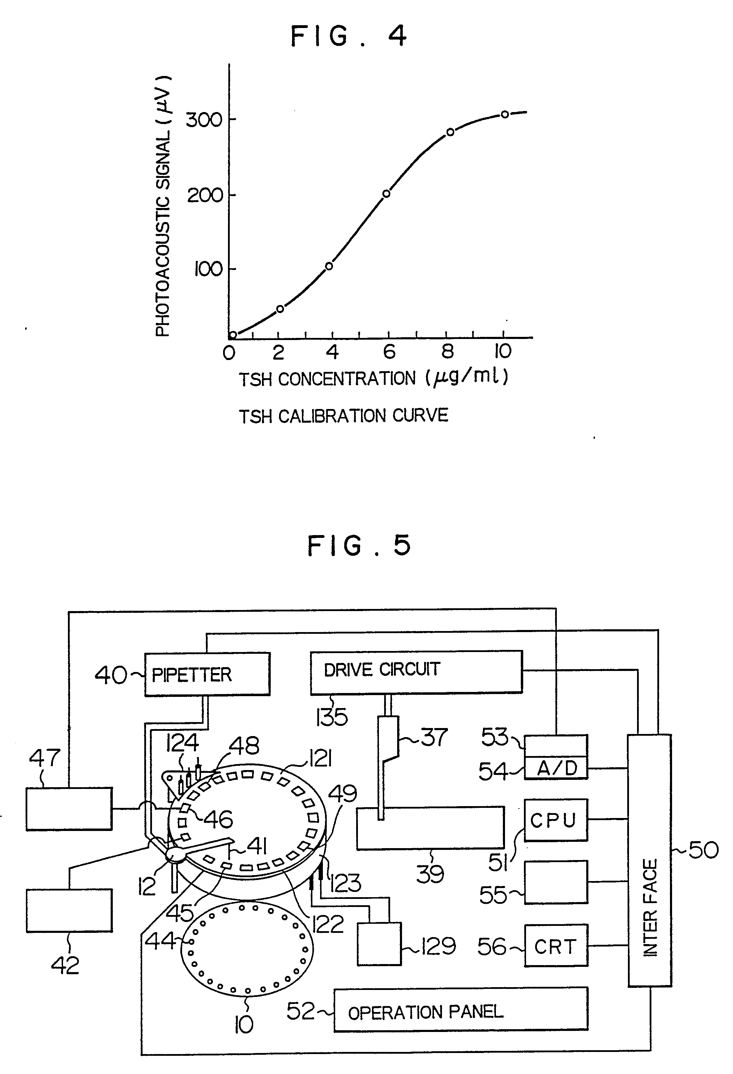

Fig. 5 shows a pretreatment equipment within the analytical apparatus in Fig. 1. The pretreatment equipment includes the reactor 21, the reagent dispenser 22 and the sample pipetter 36 in Fig. 1. A pipetter 40 in Fig. 5 corresponds to the pipetter 36 in Fig. 1. Distributor 37 in Fig. 5 corresponds to the dispenser 22 in Fig. 1.

In Fig. 5, a sample table 10 on which a plurality of the standard substances having different concentrations are placed for respective items for measurement is provided. A plurality of standard substances can be placed on the sample table 10 continuously for every item for measurement. A reaction table 121 has reactors 122, on the circumference of which the reacted solid phase 5 having bound thereto a plurality of antibodies for analyses of a plurality of items is placed. The reaction table is constructed to be freely rotatable. Transfer of the standard material and sample is performed by means of a sampling probe 41. Dispense of the reagent is effected by a moving distributor 37.

The reactors 122 in which a plurality of the solid phases in the kind and number are encased are constructed to make continuous line on the reaction table 121 for every item. A necessary solid phase is supplied to the corresponding reactor through a reacted solid phase supplier 42. On the reactor line, a discharge apparatus 129 and a washing apparatus 124 are placed. Details of the photoacoustic signal measuring apparatus 49 are shown in Fig. 1. A sealed type photoacoustic cell 23 is so constructed that a pressure change generated as the result of the exposure of the solid phase placed within the cell to the light source 17 can be detected by means of a microphone.

A controlling apparatus is equipped with a multiplexer 53, an A/D converter 54, a read only memory (hereafter referred to as ROM), a random access memory (hereafter referred to as RAM), a printer 55, an operation panel 52 and a driving circuit for mechanism 135. The A/D converter 54 is further connected to a central processor 51 via an interface 50. The central processor 51 functions to control the whole apparatus including the mechanism, prepare a calibration curve and perform data processing such as operation of the concentration, etc. For the central processor, a microcomputer is used.

The operation of the pretreatment equipment is described below.

Firstly, when a plurality of the solid phases are set on the reacted solid phase supplier 42, necessary numbers of the solid phase are continuously supplied to the reactors 122 on the reaction table 121 for every item. Next, sample containers 44 in which sera (samples) to be analysed containing hormones, cancer markers, infection-associated substances, etc. are encased are supplied on the sampling position on the sample table 10. Then, the tip of the probe 41 held in a movable arm 12 in the pipetter 40 is dipped in the sample containers and sucks a definite amount of the serum and holds in the probe 41. Thereafter, the probe 41 moves to the discharge position 45 on the reaction table 121 and discharges the serum held in the reactor 122 including the solid phases of kinds corresponding to the items to be measured, with the probe 41.

When this sampling operation is completed, the reaction table 121 goes counterclockwise by 1 pitch of the reactor and stops at the position. When a time from the rotation to the stop of the reaction table is, for example, 20 seconds, the above operation is repeated as one cycle for 20 seconds. As the cycle proceeds, the specific sample to be measured goes counterclockwise by one pitch of the reactor at the position where the reaction table 121 is in a standstill state. Discharge of the reagent from the moving distributor 37 is made in such a state that the reactor 122 stops at the discharge position 47 on the reaction table 121. The distributor 37 selects a necessary reagent solution from a line of reagent containers 39 to discharge into the reactor. Taking as an example a specific sample, a first reaction is initiated between the sample added at the discharge position 45 and the solid phase in the reactor and a second reaction is initiated with the reagent at the discharge position 47.

When the reactor 122 in which the reaction proceeds stops at a washing position 48, the discharge and suction of reagent for washing are repeated by a washing apparatus 124, whereby the solid phase is washed as it stands in the reactor. When the reaction table 121 rotates and stops at the stop position 46, only the reacted solid phase in the reactor is taken into the photoacoustic cell in which the photoacoustic signal is detected by means of a photoacoustic apparatus 49. In output of the photoacoustic apparatus 49, a signal of measurement wavelength currently required is chosen by the multiplexer 53, incorporated into the central processor 51 through the A/D converter 54, and memorized on RAM. The central processor operates following the program of ROM, extracts the measurement data in RAM and performs data processing. The reaction table 121 is placed in a thermostat 123, whereby the reaction in the reactor can be proceeded at a controlled temperature.

For example, 5 or 6 standard substances per one item which are required for preparing a calibration curve are aligned in a continuous line on the sample table 10. Accordingly, a plurality of the standard substances having different concentrations for a specific item are automatically transferred to the reaction table 121 by a plurality of times (for example, by twice each) through the sampling probe 41. In the case of preparing a calibration curve on a sample which has no linear relationship with its concentration, it is indispensable to perform sampling the standard substances having different concentrations by a plurality of times and measure them. The present apparatus enables to prepare such calibration curve. These measurement data are collected for each item and provided for the preparation of calibration curve. The measurement results are displayed on the printer 55 and CRT 56. Next, in order to verify the effects of the present invention, an example of analyzing a serum sample by appropriately modifying the apparatus of Fig. 1 is shown below.

Measurement Example 1

A removable rotary holder was used instead of the reaction table 121 shown in Fig. 5. A plurality of solid phase disks composed of a porous material capable of filtering liquid therethrough were set on the holder. After the cellulose membrane having bound thereto anti-human HCG (human chorionic gonadotropin) antibody was adsorbed to the porous reacted solid phase disks, the holder on which the disks were placed was put on the reaction apparatus 21. Through the sample pipetter 36, 100 $\mu$l of the serum sample was dispensed on the reacted solid phase disks. Five minutes after, 100 $\mu$l of anti-human HCG antibody solution labeled with FITC (fluorescein isothiocyanate) was added to the solid phase disks and superfluous water was absorbed into the porous material. After allowing to stand at room temperature, Tris-hydrochloride buffer solution (pH 7.8) was added to the solid phase disk. By doing so, the unreacted excess FITC-labeled anti-human HCG antibody and water were absorbed into the porous material to remove them from the cellulose membrane surface. The antigen-antibody complex was formed on the cellulose membrane of the solid phase disk.

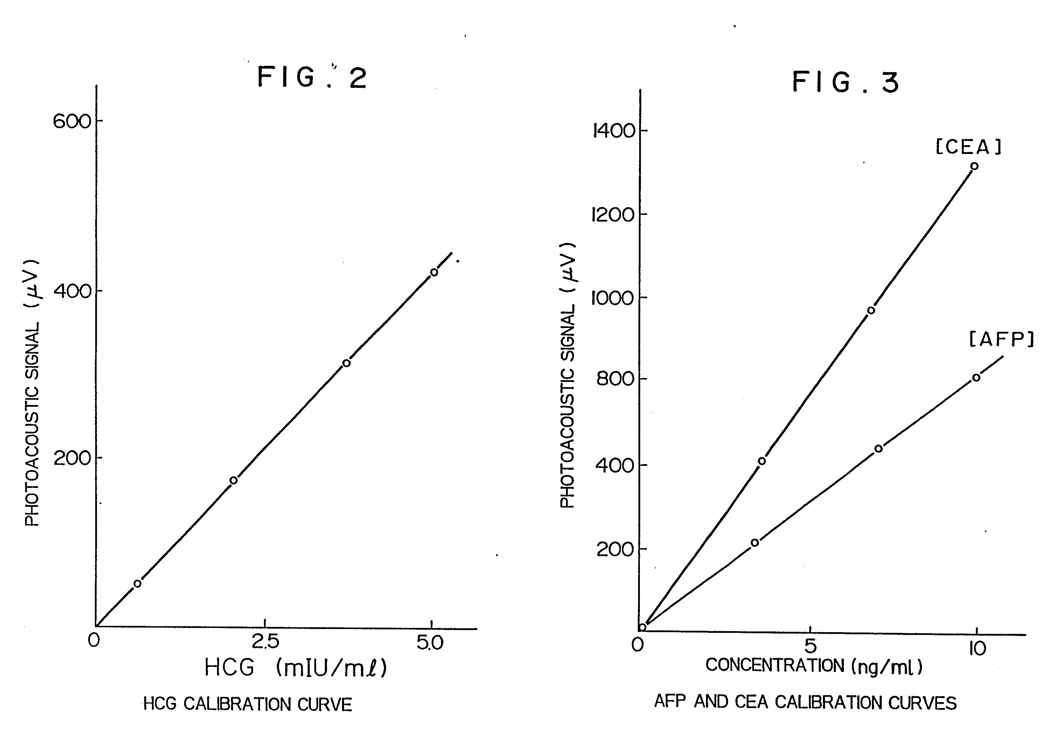

Next, the reacted solid phase in the reaction apparatus 21 was transferred to the photoacoustic cell 23 by means of the reacted solid phase transfer 20. The photoacoustic cell was covered with a cover for sealing. A laser light from the light source 17 was then cast into the cell 23 to measure the photoacoustic signal based on the antigen-antibody complex formed on the cellulose membrane surface. Thus, HCG in the sample was measured; an example of calibration curve in this case is shown in Fig. 2.

Measurement Example 2

Filter paper (Toyo No. 51) was cut into a size of 1 x 2 cm and made a raw material for the solid phase. About 1 g of the paper solid phase was suspended in 20 ml of 5 M potassium phosphate buffer (pH 12.1) and 10 ml of water was added to the suspension. While stirring with a stirrer, 10 ml of BrCN solution (0.1 g/l) was gradually added to the mixture over 2 minutes followed by reacting for further 6 minutes. The reaction was carried out at 4°C. Immediately after the reaction, the reaction mixture was washed with a large quantity of 0.005 M ice-cooled $NaHCO_3$ solution, and 5 mg of purified anti-human AFP antibody and purified anti-human CEA antibody were reacted at room temperature for 8 hours. After further reacting with 1 M ethanolamine (pH 8.0) at 4°C overnight, the reaction mixture was sequentially washed with 0.5 M $NaHCO_3$ solution, 0.1 M acetate buffer solution (pH 4.0) and 0.015 M PBS (pH 7.2), and then freeze dried in 1.5 ml of PBS (pH 7.2).

After the solid phase having bound thereto anti-human AFP antibody and anti-human CEA (carcino-embryonic antigen) antibody was set on the reaction apparatus 21, 100 $\mu$l of a serum sample was added to the solid phase. Immune reaction was carried out at room temperature for 10 minutes. The solid phase was then washed twice with 1 ml each of 0.9% NaCl and the washing liquid was removed from the system.

A first reagent dispersion of yellow latex particles having bound thereto anti-human AFP antibody and a second reagent dispersion of blue latex particles having bound thereto anti-human CEA antibody were prepared as the reagents. To the solid phase were added 250 $\mu$l each of the first and second reagent dispersions. The mixture was reacted at room temperature for 10 minutes. The reaction mixture was then washed twice with 1 ml of 0.9% NaCl. After the washing liquid was removed, the solid phase was withdrawn from the reaction apparatus 21 and placed in the photoacoustic cell 23. After the cell 23 was sealed, the solid phase was exposed to 2 kinds of laser light having different wavelengths, whereby the photoacoustic signal was measured. According to such a method, AFP and CEA can be quantitatively determined concurrently on one reacted solid phase disk. An example of the calibration curve for simultaneous quantitative determination of AFP and CEA used in this measurement method is shown in Fig. 3. In this case, AFP and CEA are measured using the wavelength corresponding thereto.

Measurement Example 3

As the solid phase, a disk-like glass piece was used. Anti-TSH (thyroid stimulating hormone) antibody was previously bound to the glass wall surface on the solid phase. The reactor in which the solid phase was encased was placed on the reaction apparatus 21. Through the sample pipetter 36, 20 $\mu$l of the serum sample containing TSH was dispensed in the reactor followed by reacting with the anti-TSH antibody on the solid phase at 37°C for 5 minutes.

Next, 100 $\mu$l of a dispersion of sheepanti-rabbit IgG antibody-bound red latex particles was added to the reaction mixture through the reagent dispenser 22 followed by reacting at 37°C for 5 minutes. Then, the unreacted liquid was removed. After washing with 0.05 M phosphate buffer solution (pH 7.5), the washing liquid was discharged. The washed solid phase was introduced into the photoacoustic cell 23 and the cell 23 was sealed. A laser light was then cast onto the solid phase to measure the photoacoustic signal. An example of calibration curve for quantitatively determining TSH is shown in Fig. 4.

## Claims

1. A method for immunoassay of an antigen-antibody complex by photoacoustic spectroscopy which comprises:
immunoreacting an antigen or antibody labeled with a color material in liquid to immobilize the labeled antigen-antibody complex on a solid phase;
separating the complex-immobilized solid phase from the liquid in contact therewith; and,
transferring the solid phase separated from the liquid to the position for exposure to light and measuring the photoacoustic property based on the color material.

2. A method for immunoassay according to claim 1, wherein said solid phase has a surface capable of holding the liquid.

3. A method for immunoassay according to claim 2, wherein said solid phase is film-like or plate-like.

4. A method for immunoassay according to claim 1, wherein said solid phase is capable of being put in a measuring cell and said solid phase is provided for photoacoustic spectroscopy in such a state that a gas is sealed as a pressure medium in said measuring cell.

5. A method for immunoassay according to claim 1, wherein said color material is selected from a fluorescent substance, a dye and colored particles.

6. A method for immunoassay according to claim 1, wherein said color material is composed of microcapsules in which a dye or fluorescent substance is encapsulated.

7. A method for immunoassay according to claim 1, wherein said antigen-antibody complex is labeled with a plurality of color materials.

8. A method for immunoassay which comprises:
adsorbing a first antibody onto a reacted solid phase;
reacting said first antibody with an antigen in a sample on said reacted solid phase to form the antigen-antibody complex on said reacted solid phase;
immobilizing a second antibody labeled with a colored label on said reacted solid phase;
removing the liquid on the reacted solid phase and introducing the reacted solid phase into a photoacoustic cell; and,
subjecting the reaction product on said reacted solid phase to photoacoustic spectroscopy.

9. A method for immunoassay which comprises:
reacting a first antibody immobilized on a solid phase with an antigen in a sample to form a antigen-antibody complex on said solid phase;
labeling a second antibody capable of specifically reacting with said first antibody with a color label and reacting the labeled second antibody with the first antibody on said solid phase; and,
then introducing the reaction product-immobilized on the solid phase into a measuring cell to perform a photoacoustic spectroscopy.

# FIG. 1

EP 0 369 176 A2

FIG. 2

HCG CALIBRATION CURVE

FIG. 3

AFP AND CEA CALIBRATION CURVES

EP 0 369 176 A2

## F I G . 4

TSH CALIBRATION CURVE

## F I G . 5

# FIG. 6